# EUROPEAN PATENT APPLICATION

(11) **EP 2 711 419 A1**
(43) Date of publication of application: **26.03.2014**
(21) Application number: 12784895.0
(22) Date of filing: 14.05.2012
(51) Int. Cl.: C12N 5/0793, A61K 31/166, A61K 31/203, A61K 31/235, A61K 31/277, A61K 31/426, A61P 25/00, A61P 43/00

(54) **CELL DIFFERENTIATION INDUCER AND DIFFERENTIATION INDUCING METHOD**

(30) Priority: 19.05.2011 JP 2011112831
(71) Applicant: The University of Tokushima, Tokushima-shi Tokushima 770-8501 (JP)
(72) Inventor: TSUJI, Daisuke, Tokushima-shi Tokushima 770-8505 (JP); ITOH, Kohji, Tokushima-shi Tokushima 770-8505 (JP); SANO, Shigeki, Tokushima-shi Tokushima 770-8505 (JP); NAKAO, Michiyasu, Tokushima-shi Tokushima 770-8505 (JP)
(74) Representative: Müller-Boré & Partner Patentanwälte PartG mbB
(86) International application number: PCT/JP2012/062318
(87) International publication number: WO 2012/157612

(57) **Abstract**

The objective of the present invention is to provide a cell differentiation inducer which is a low-molecular compound, which can be chemically synthesized easily and which can efficiently induce a differentiation of an undifferentiated cell into a nervous system cell with high selectivity. In addition, the objective of the present invention is to provide use of a specific catechol derivative for efficiently inducing a differentiation of an undifferentiated cell into a nervous system cell with high selectivity, and a method for efficiently inducing a differentiation of an undifferentiated cell into a nervous system cell with high selectivity using a specific catechol derivative. The cell differentiation inducer of the present invention is characterized in comprising the catechol derivative having the specific chemical structure.

## Description

### TECHNICAL FIELD

The present invention relates to a cell differentiation inducer containing a catechol derivative having a low molecular weight, use of the catechol derivative for inducing a differentiation of an undifferentiated cell, and a method for inducing a differentiation of an undifferentiated cell into a nervous system cell such as a neural crest cell using the catechol derivative.

### BACKGROUND ART

A cell which constitutes a multicellular organism is roughly classified into a differentiated cell, a TA cell: Transient Amplifying Cell and a stem cell. A differentiated cell is also called as a terminally differentiated cell and exemplified by a nerve cell and an organ cell. A differentiated cell does not further become differentiated into a different cell and hardly grow. A TA cell has a property intermediate between a differentiated cell and a stem cell, and after differentiation, actively proliferates to be a differentiated cell. A stem cell is defined as a cell which has both of abilities to autonomously proliferate and be differentiated, and can autonomously proliferate and be differentiated into a TA cell.

In other words, a stem cell can undergo differentiation into any cells. For example, a stem cell can become differentiated into a totipotent cell, which has the potential to grow into an individual, a unipotent cell, which can undergo differentiation into a specific cell only, and a pluripotent cell, which does not have the potential to grow into an individual without a specific procedure but can undergo differentiation into various cell. A totipotent cell is exemplified by a fertilized egg. A unipotent cell is exemplified by a germ stem cell such as a sperm. A pluripotent cell is exemplified by a pluripotent stem cell.

By using a stem cell, for example, it may become possible that a dermal tissue or an organ tissue is prepared from a patient himself/herself. As a result, a regenerative medical treatment without rejection may be possible. In addition, offending cells may be proliferated from a patient of a specified disease such as a genetic disease and the proliferated cells may be utilized for developing a new drug.

In particular, a technology to artificially prepare a pluripotent undifferentiated cell such as an ES cell and an iPS cell was developed. As a result, the application of the technology to a regenerative medicine and a drug design research has been expected. It is therefore thought that the technology to induce a differentiation of an undifferentiated cell into a desired functional cell may be required hereafter.

It is preferred to differentiate a pluripotent cell such as an ES cell specifically into a particular cell, since when a cell is disorderly differentiated, the use for a desired purpose may possibly become difficult, for example, it may become necessary to select a kind of a differentiated cell. For example, in Patent Document 1, a sesamin derivative is disclosed as a differentiation inducer to a nerve cell. In addition, in Patent Document 2 and Non-patent Document 1, retinoic acid is disclosed as a differentiation inducer from an undifferentiated cell into an ectodermal cell.

An early embryo of a multicellular organism is classified into an ectoderm, a mesoderm and an endoderm. An ectoderm becomes nerve, a dermal tissue and the like, a mesoderm becomes muscle, bone, blood vessel, blood and the like, and an endoderm becomes a specific organ such as the digestive tract and the lung. In addition, there is a neural crest cell. A neural crest cell is fallen into the category of an ectoderm; but is also referred to as the forth germ layer, since a neural crest cell play a specific and important role. For example, a neural crest cell migrates from neural crest in neurula stage, and becomes a melanocyte, a peripheral nervous neuron, smooth muscle, a cartilage and bone mainly in head.

As a conventional differentiation induction method from an undifferentiated cell such as an ES cell into a neural crest cell, SDIA method has been known (Non-patent Document 2). In SDIA method, ES cells are added to a serum-free culture medium containing feeder cells, and BMP4: Bone Morphogenetic Protein 4, which is a bone morphogenetic protein, is added thereto. In addition, a method for inducing a differentiation of an undifferentiated cell into a neural crest cell using a culture medium containing specific components and without using a feeder cell is disclosed in Patent Document 3.

### PRIOR ART

### PATENT DOCUMENT

Patent Document 1: JP 2010-202559 A
Patent Document 2: JP 2007-535957 T
Patent Document 3: WO 2010/140698

### NON-PATENT DOCUMENT

Non-Patent Document 1: Bain, G et al., Developmental Biology, 168, pp.342-357 (1995)
Non-Patent Document 2: Kenji MIZUSEKI et al., Proceedings of the National Academy of Science of the United States of America, 100, pp.5828-5833 (2003)

### DISCLOSURE OF THE INVENTION

### PROBLEMS TO BE SOLVED BY THE INVENTION

As described above, a method for inducing a differentiation of an undifferentiated cell such as an ES cell into a neural crest cell has been known. However, in SDIA method described in Non-patent Document 2, a feeder cell is used. Therefore, there is a problem that even if a neural crest cell which is induced from an iPS cell derived from a patient himself/herself is used, rejection may cause when the neural crest cell is introduced into the patient. In addition, there is also a problem that a differentiation-inducing ratio may largely changes depending on the lot of the used feeder cell. Further, since BMP4, which is a secretory protein, is very expensive, there is a problem of high cost when BMP4 is used as a cell differentiation inducer. Although a feeder cell is not needed in the method described in Patent Document 3, there are also problems of high cost and low efficiency since a differentiation-inducing ratio is very low when a secretory protein such as BMP4, which is a bone morphogenetic protein, and the like are not used.

On the other hand, a sesamin derivative used in the invention of Patent Document 1 is a low-molecular compound; however, in the invention, a differentiation of a precursor cell of a nerve cell is merely induced into a nerve cell and a differentiation of an iPS cell is not induced. In addition, even though a sesamin derivative is a low-molecular compound, a sesamin derivative must be extracted from sesame oil and is difficult to be chemically synthesized due to four asymmetric carbons. Further, retinoic acid described in Patent Document 2 and Non-patent Document 1 is also a low-molecular compound; however, retinoic acid is not a good cell differentiation inducer since when an ES cell is treated with retinoic acid, a differentiation into cells other than an ectodermal cell are induced.

As described above, there is conventionally no cell differentiation inducer which is an inexpensive low-molecular compound and which can selectively induce of a differentiation of an undifferentiated cell into a nervous system cell with high efficiency.

Under the above-described circumstance, the objective of the present invention is to provide a cell differentiation inducer which is a low-molecular compound, which can be chemically synthesized easily and which can efficiently induce a differentiation of an undifferentiated cell into a nervous system cell with high selectivity. In addition, the objective of the present invention is to provide use of a specific catechol derivative for efficiently inducing a differentiation of an undifferentiated cell into a nervous system cell with high selectivity, and a method for efficiently inducing a differentiation of an undifferentiated cell into a nervous system cell with high selectivity using a specific catechol derivative.

### MEANS FOR SOLVING THE PROBLEMS

The inventors of the present invention intensively studied for solving the above problem. As a result, the inventors completed the present invention by finding that a specific catechol derivative can efficiently induce a differentiation of an undifferentiated cell into a nervous system cell though the catechol derivative has very simple chemical structure.

The cell differentiation inducer according to the present invention is characterized in comprising a catechol derivative represented by the following formula (I): 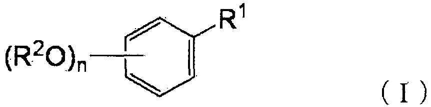 wherein
R¹ is a carboxy group, a (C₁₋₆ alkoxy) carbonyl group, a C₁₋₇ alkanoyl group, a C₂₋₈ alkynyl group, a carbamoyl group, a cyano group, a nitro group, a halogen atom, an oxazolyl group optionally-having a substituent α, a thiazolyl group optionally-having a substituent α, an oxazolinyl group optionally-having a substituent α, or a thiazolinyl group optionally-having a substituent α;
R² is a hydrogen atom, a C₁₋₆ alkyl group or a benzyl group;
n is an integer of not less than 2 and not more than 5;
substituent α is one or more groups selected from a carboxy group, a (C₁₋₆ alkoxy) carbonyl group, a C₁₋₇ alkanoyl group, a carbamoyl group, a cyano group, a nitro group and a halogen atom;
provided that two or more R²O groups may be the same or different from each other.

In the present invention, a "C₁₋₆ alkyl group" is a linear or branched saturated aliphatic hydrocarbon group having 1 to 6 carbon atoms. The group is exemplified by a methyl group, an ethyl group, an n-propyl group, an isopropyl group, an n-butyl group, an isobutyl group, a t-butyl group, an n-pentyl group and an n-hexyl group. The group is preferably a C₁₋₄ alkyl group, more preferably C₁₋₂ alkyl group, and most preferably a methyl group.

The term " (C₁₋₆ alkoxy) carbonyl group" is a (C₁₋₆ alkyl-O-C(=O)-group. The group is exemplified by a methoxycarbonyl group, an ethoxycarbonyl group, an n-propoxycarbonyl group, an isopropoxycarbonyl group, an n-butoxycarbonyl group, an isobutoxycarbonyl group, a t-butoxycarbonyl group, an n-pentoxycarbonyl group and an n-hexoxycarbonyl group. The group is preferably a (C₁₋₄ alkoxy) carbonyl group, more preferably a (C₁₋₂ alkoxy) carbonyl group, and most preferably a methoxycarbonyl group.

The term "C₁₋₇ alkanoyl group" means a formyl group and a carbonyl group substituted with the above-described C₁₋₆ alkyl group. The group is exemplified by a formyl group, an acetyl group, an n-propionyl group, an isopropionyl group, an n-butyryl group, an isobutyryl group, a pentanoyl group, a pivaloyl group, a valeryl group and an isovaleryl group. The group is preferably a C₁₋₅ alkanoyl group, more preferably C₁₋₃ alkanoyl group, and most preferably a formyl group or an acetyl group.

The term "C₂₋₈ alkynyl group" is a linear or branched unsaturated aliphatic hydrocarbon group which has carbon-carbon triple bond and which has 2 to 8 carbon atoms. It is preferred that the 1 position carbon, which is bound to the benzene ring of the catechol derivative (I), of the group has a carbon-carbon triple bond. The group is exemplified by an ethynyl group, a 1-propynyl group, a 1-butynyl group, a 1-pentynyl group, a 3-methyl-1-butynyl group and a 4-methyl-1-pentynyl group. The group is preferably a C₂₋₄ alkynyl group, and more preferably an ethynyl group and 1-propynyl group.

The term "halogen atom" is exemplified by a fluorine atom, a chlorine atom, a bromine atom and an iodine atom, preferably a chlorine atom, a bromine atom or an iodine atom, and more preferably a chlorine atom or a bromine atom.

With respect to the cell differentiation inducer of the present invention, it is preferred that R² is a hydrogen atom, two or more R²O groups are adjacent to each other, and R¹ is a cyano group or a carbamoyl group. According to the experimental findings by the inventors, the above cell differentiation inducers can particularly induce a differentiation of an undifferentiated cell into a nervous system cell with relatively low concentration, high selectivity and high efficiency.

In addition, the "n" is preferably an integer of not less than 2 and not more than 4, and more preferably 2 or 3. In general, a benzene compound which has many substituents is more difficult to be synthesized and more expensive.

Two or more R²O groups may be the same or different from each other. It is preferred that two or more R²O groups are the same, since the compound (I) is easier to be synthesized in such a case.

When the compound (I) has a carboxy group as a substituent, the compound may be a salt. When the neural crest cell obtained by the present invention is used for regenerative medicine, it is preferred that the salt is pharmaceutically acceptable. Such a salt is exemplified by an alkali metal salt such as a sodium salt and a potassium salt; an alkaline earth metal salt such as a calcium salt and a magnesium salt; an inorganic amine salt such as an ammonium salt; and an organic amine salt such as a trimethylamine salt, a triethylamine salt and a pyridine salt.

The number of substituent α of the thiazolyl group is not particularly limited as long as the substituent can be substituted, and is preferably not less than 1 and not more than 2, and more preferably 1.

It is preferred that the cell differentiation inducer of the present invention further contains retinoic acid. Effect to induce an undifferentiated cell into a nervous system cell is further improved by the combination with retinoic acid.

It is preferred that the cell differentiation inducer of the present invention is used for inducing a differentiation of an undifferentiated cell to a nervous system cell.

The method for inducing a differentiation of an undifferentiated cell to a nervous system cell is characterized in comprising the step for treating an undifferentiated cell with the cell differentiation inducer of the present invention.

The catechol derivative (I) of the present invention is the active ingredient of the above-described cell differentiation inducing agent, and used for inducing differentiation of an undifferentiated cell.

The method for inducing a differentiation of an undifferentiated cell into a nervous system cell according to the present invention is characterized in comprising the step for cultivating an undifferentiated cell in a culture medium containing the catechol derivative (I), which is the active ingredient of the above-described cell differentiation inducing agent.

### EFFECT OF THE INVENTION

The catechol derivative (I), which is the active ingredient of the cell differentiation inducing agent according to the present invention, is available at a low price or can be easily synthesized, since the catechol derivative has a very simple chemical structure. Therefore, the catechol derivative is lower in cost than expensive protein. In addition, the catechol derivative (I), which is the active ingredient of the cell differentiation inducing agent according to the present invention, can induce a differentiation of an undifferentiated cell into a nervous system cell with high selectivity and good efficiency without using a feeder cell even at a relatively low concentration. Therefore, the present invention is industrially very useful, since it may be facilitated by the present invention to realize regenerative medicine, drug discovery research or the like by using an iPS cell.

### BRIEF DESCRIPTION OF THE DRAWING

Figure 1 is a magnified photograph of the case that a mouse ES cell was treated with the cell differentiation inducer of the present invention and a control magnified photograph of the case that a mouse ES cell was similarly treated except for not using the cell differentiation inducer of the present invention.
Figure 2 is a magnified photograph of the case that a mouse ES cell was treated with the cell differentiation inducer of the present invention.
Figure 3 is a magnified photograph of the case that a mouse ES cell was treated with the cell differentiation inducer of the present invention.
Figure 4 is a magnified photograph of the case that a differentiation of a mouse ES cell was induced using the cell differentiation inducer of the present invention or retinoic acid and the induced cells were stained using an ectodermal stem cellular marker and a mesodermal stem cellular marker.
Figure 5 is an electrophoresis photograph to demonstrate the result of analyzing the gene expression in a mouse ES cell, the germ layer thereof, and the mouse ES cell which was treated with the cell differentiation inducer of the present invention.
Figure 6 is a magnified photograph of the case that a human iPS cell was treated with the cell differentiation inducer of the present invention and a control magnified photograph of the case that a human iPS cell was similarly treated except for not using the cell differentiation inducer of the present invention.
Figure 7 is a graph to demonstrate the combination effect of the cell differentiation inducer according to the present invention and retinoic acid.
Figure 8 is a magnified photograph of the case that a mouse ES cell was treated with the cell differentiation inducer of the present invention.

### MODE FOR CARRYING OUT THE INVENTION

The cell differentiation inducer of the present invention contains the catechol derivative represented by the formula (I). The catechol derivative (I) has a very simple chemical structure. Therefore, the catechol derivative may be purchased, if the derivative is commercially available. Alternatively, the person skilled in the art can easily synthesize the catechol derivative from a commercially available compound by an ordinary method.

For example, various benzene compounds which have two or more hydroxy groups are commercially available. The person skilled in the art can transform such a phenolic hydroxy group into an alkoxy group or a benzyloxy group.

In addition, the person skilled in the art can introduce R¹ group depending on a substitution position of the phenolic hydroxy group and carry out functional group transformation reaction. For example, the following functional group transformations are possible. 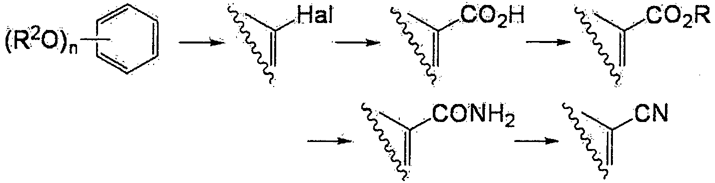 In the above formulae, "Hal" is a halogen atom and R is C₁₋₆ alkyl group.

Further, the compound (I) which has an oxazolinyl group or a thiazolinyl group can be synthesized by reacting a benzonitrile compound with serine or cysteine for ring-closing as the following reaction formula. In this case, an absolute configuration at a carbon atom at which a carboxy group is substituted in an oxazolinyl ring or a thiazolinyl ring can be controlled by using optically-active serine or cysteine. In addition, a carboxy group in an oxazolinyl ring or a thiazolinyl ring can be subjected to functional group transformation as the above. Hereinafter, a reaction to form a thiazolinyl group by using cysteine is demonstrated as a typical example.

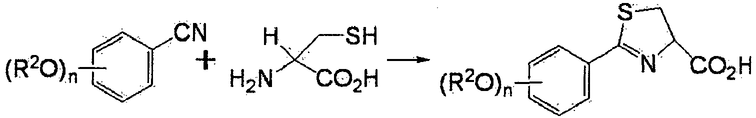

In the above reaction, a benzonitrile compound is reacted with cysteine in the presence of abase. It is preferred to use a mixed solvent of water and a water-miscible organic solvent as a solvent, since cysteine or serine can be dissolved in water but some benzonitrile compounds are hardly dissolved in water. Such a water-miscible organic solvent is exemplified by an alcohol solvent such as methanol and ethanol; an amide solvent such as dimethylformamide and dimethylacetamide; a sulfoxide solvent such as dimethylsulfoxide. In addition, a buffer solution may be used as water for maintaining pH of the reaction mixture. As a base, a hydrogencarbonate salt of an alkali metal, such as sodium hydrogencarbonate; a carbonate salt of an alkali metal, such as sodium carbonate; a hydroxide of an alkali metal, such as sodium hydroxide; and the like may be used. A reaction temperature is not particularly limited and may be arbitrarily adjusted, and is usually adjusted to not less than 30°C and not more than 100°C.

As the following reaction formula, the compound (I) which has an oxazolyl group or a thiazolyl group can be synthesized by reacting a benzamide compound or a thiobenzamide compound with a halogenated pyruvate ester compound and cyclizing the obtained compound. The alkoxycarbonyl group, i.e. the ester group, of the oxazolyl ring or thiazolyl ring can be subjected to functional group transformation similarly as the above. Hereinafter, a reaction to form a thiazolyl group by using the thiobenzamide compound is demonstrated as a typical example.

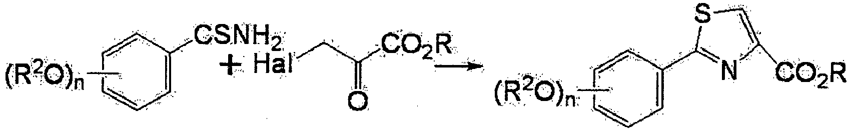

In the above formula, "Hal" is a halogen atom, and R is a C₁₋₆ alkyl group, particularly a C₁₋₂ alkyl group.

In the above reaction, the thiobenzamide compound is reacted with the halogenated pyruvate ester compound. A solvent to be used is exemplified by an alcohol solvent such as methanol and ethanol; a halogenated hydrocarbon solvent such as dichloromethane and chloroform; and an amide solvent such as dimethylformamide and dimethylacetamide. A reaction temperature is not particularly limited and may be arbitrarily adjusted, and is usually adjusted to not less than 30°C and not more than 100°C.

The catechol derivative of the present invention may further include retinoic acid. When retinoic acid is used in combination, an inducing effect into a nervous system cell is further improved.

A content of retinoic acid may be arbitrarily adjusted, and for example, it is preferred to use not less than 0.5 times by mole and not more than 2.0 times by mole relative to 1 mole of the catechol derivative of the present invention. When the ratio is not less than 0.5 times by mole, a synergistic effect by retinoic acid can be expected. On the other hand, when the ratio is too high, selectivity may possibly decreased since a differentiation into a cell other than an ectodermal cell is induced by retinoic acid; therefore, the ratio is preferably not less than 2.0 times by mole. The ratio is more preferably not less than 0.7 times by mole, even more preferably not less than 0.8 times by mole, and more preferably not more than 1.5 times by mole, even more preferably not more than 1.2 times by mole.

As described above, the catechol derivative which is a main component of the cell differentiation inducer according to the present invention has a very simple chemical structure and can be synthesized very easily, but a differentiation of an undifferentiated cell can be effectively induced into a nervous system cell with high selectivity by using the catechol derivative. Hereinafter, the method for inducing a differentiation is described.

### (1) Preparation of undifferentiated cell

An undifferentiated cell used in the present invention method is not particularly limited as long as the cell has pluripotency to differentiate into a nervous system cell. For example, an undifferentiated cell which has an ability to differentiate into a nervous system cell and self-renew, such as an ES cell, an iPS cell and a mesenchymal stem cell, is particularly useful for applying to regenerative medicine. In addition, an embryoid which is obtained from the above-described cells is included in the range of the undifferentiated cell used in the present invention. When an embryoid is used, it becomes easier to induce a differentiation into a nervous system cell, since an ectoderm, a mesoderm and an endoderm are generally included in an embryoid and a nervous system cell is an ectodermal cell.

The origin of an undifferentiated cell is not particularly limited, and may be derived from fish, an amphibian, a bird or a mammalian. It is preferred that an undifferentiated cell is derived from a mammalian considering an application to a regenerative medicine, and it is more preferred that an undifferentiated cell is derived from a human.

An undifferentiated cell may be prepared in a usual manner. For example, an ES cell can be isolated as an undifferentiated stem cellular mass by cultivating inner clump of cells which exist inside of blastocyst-stage embryo in vitro and repeating dissociation and passage of the cellular mass. An iPS cell can be prepared by transferring specific genes into a somatic cell such as a fibroblast cell. A mesenchymal stem cell can be isolated from cord blood, bone marrow, placenta and the like, and cultured to be used. An embryoid can be obtained by culturing the above cells in a suspension manner in a culture medium for forming an embryoid.

### (2) Addition of the cell differentiation inducer of the present invention

The above-described undifferentiated cell can be treated by adding the cell differentiation inducer of the present invention to a culture medium of the undifferentiated cell.

In particular, when an embryoid is used as an undifferentiated cell, a culture medium for forming an embryoid is preferably changed to a culture medium which is suitable for inducing a differentiation. For example, such a culture medium contains an inorganic salt such as sodium chloride, potassium chloride, calcium chloride, iron nitrate, magnesium sulfate, sodium hydrogencarbonate, sodium dihydrogenphosphate; an amino acid such as a unnatural amino acid and a natural amino acid; a vitamin such as calcium pantothenate, folic acid, inositol, nicotinamide, pyridoxine hydrochloride and thiamine hydrochloride; and other nutrients such as glucose and sodium pyruvate. In addition, a basic fibroblast growth factor (bFGF), an epidermal growth factor (EGF), a leukocyte migration inhibitory factor (LIF), serum or the like may be added.

The cell differentiation inducer of the present invention may be directly added to a culture medium for an undifferentiated cell. However, it is preferred that the cell differentiation inducer is preliminarily dissolved to be a solution, since the cell differentiation inducer may not be adequately mixed in some cases due to a problem such as insufficient solubility. When the cell differentiation inducer of the present invention can be sufficiently dissolved in water, distilled water is used as a solvent of the solution; on the other hand, when the cell differentiation inducer cannot be sufficiently dissolved in water, the cell differentiation inducer may be dissolved in a water-miscible organic solvent to be added. Such a water-miscible organic solvent is exemplified by an alcohol solvent such as methanol and ethanol; an amide solvent such as dimethylformamide and dimethylacetamide; and a sulfoxide solvent such as dimethylsulfoxide. However, since the organic solvents may possibly have an adverse effect on a cell in some cases, it is preferred that an additive amount of an organic solvent is decreased as much as possible by setting the solvent concentration to be higher.

An additive amount of the cell differentiation inducing agent according to the present invention may be appropriately adjusted depending on the activity of the compound and the like, and for example, the additive amount may be set to be not less than about 10 µM and not more than about 300 µM in the total amount of a culture medium. When the concentration is 10 µM or more, it becomes possible to promote induction of a differentiation into a neural system cell more reliably. On the other hand, when the concentration is too high, cell cytotoxicity may be possibly exhibited in some cases. Therefore, the concentration is preferably 300 µM or less. The concentration is more preferably not less than 15 µM , even more preferably not less than 20 µM, and more preferably not more than 200 µM, even more preferably not more than 150 µM.

The culture conditions after the addition of the cell differentiation inducer according to the present invention may be appropriately adjusted depending on the kind of an undifferentiated cell to be used or the like. For example, culture may be carried out under 5% carbon dioxide atmosphere at not less than about 25°C and not more than about 45°C for not less than 1 day and not more than about 10 days.

A differentiation of an undifferentiated cell into a nervous system cell can be induced by using the cell differentiation inducer of the present invention. In the present invention, a nervous system cell is exemplified by a precursor cell of a nerve cell, such as a neural stem cell, a neural precursor cell and a neural crest cell, in addition to a nerve cell such as a nerve cell and a glial cell.

It can be checked by morphological observation, identification of expressed protein, gene expression profiling or the like whether a differentiation into a nervous system cell is induced or not. For example, a general nerve cell has a characteristic morphology with cell process. In addition, a neural crest cell is an ectodermal cell, but expresses not only Nestin, which is a marker of an ectodermal stem cell, but also Flk1, which is a marker of a mesodermal stem cell. Further, a marker of a neural crest cell is exemplified by Sox9, Sox10 and Slug. It can be checked by ELISA in which an antibody specific for the above-described proteins is used as a primary antibody and expression profiling of a gene which codes the above-described proteins whether a differentiated cell is a neural crest cell or not.

As described above, a nervous system cell is specified by morphological observation and the like, and can be separated from other cells.

An isolated nervous system cell can be applied to treatment of a disease related to a neural cell, regenerative medicine or the like. For example, a neural crest cell which is differentiated by the present invention is isolated, and the isolated neural crest cell can be applied to a treatment of a disease due to a defect of induction or formation of a neural crest and a defect of neural crest cell migration, regenerative medicine or the like. In addition, the obtained neural crest cell is differentiated into a glial cell, a pigment cell, a keratocyte, a smooth muscle cell, a chondrocyte, a bone cell or the like, and the cells can be applied to a treatment of a disease related to the cells and regenerative medicine.

The present application claims the benefit of the priority date of Japanese patent application No. 2011-112831 filed on May 19, 2011, and all of the contents of the Japanese patent application No. 2011-112831 filed on May 19, 2011, are incorporated by reference.

### EXAMPLES

Hereinafter, the present invention is described in more detail with Examples. However, the present invention is not limited by the following Examples, and the present invention can be appropriately modified to be carried out within a range which adapts to the contents of this specification. Such a modified embodiment is also included in the range of the present invention.

### Example 1: 2-{2,3-Bis(benzyloxy)phenyl}thiazole-4-carboxylic acid

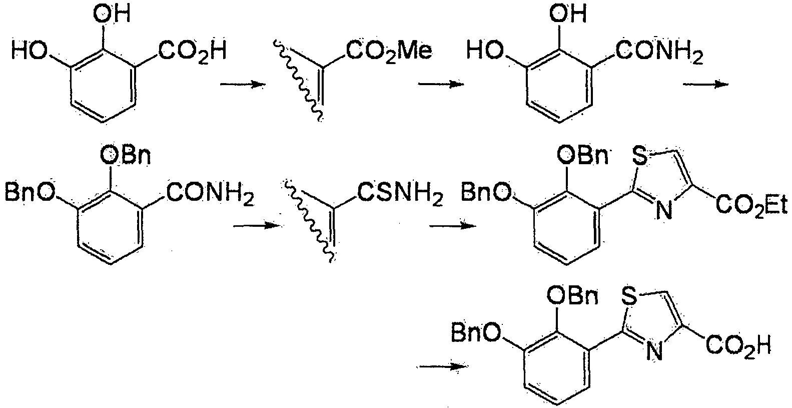

### (1) 2,3-Dihydroxybenzamide

In methanol (50mL), 2,3-dihydroxybenzoic acid (5g, 32.4 mmol) was dissolved. The obtained solution was cooled to 0°C. Concentrated sulfuric acid (3 mL) was added dropwise thereto at the same temperature, and the mixture was heated to reflux for 15 hours. The reaction mixture was concentrated under reduced pressure. A saturated aqueous solution of sodium hydrogencarbonate was added thereto until foaming did not occur, and then extraction was carried out three times using ethyl acetate (100 mL). The organic layer was dried using anhydrous magnesium sulfate, and filtrated. The obtained filtrate was concentrated under reduced pressure. To the residue, 28% aqueous ammonia (92 mL) was added, and the mixture was stirred at 50°C for 16 hours. Then, the reaction mixture was concentrated to about 20 mL under reduced pressure, and extraction was carried out five times using ethyl acetate (50 mL). The organic layer was dried using anhydrous magnesium sulfate, and filtrated. The obtained filtrate was concentrated under reduced pressure, to obtain the target compound as a pale pink solid (3.92 g, yield: 80%).

### (2) 2,3-Bis(benzyloxy)benzamide

In dimethylformamide (13.06 mL), 2,3-dihydroxybenzamide(2 g, 13.06 mmol) was dissolved. Potassium carbonate (9.03 g, 65 mmol) and benzyl bromide (3.26 mL, 27.41 mmol) were added to the obtained solution at room temperature, and the mixture was stirred for 18 hours. Then, water (20 mL) was added to the reaction mixture, and then extraction was carried out three times using ethyl acetate (100 mL). The organic layer was dried using anhydrous magnesium sulfate, and filtrated. The obtained filtrate was concentrated under reduced pressure to obtain a crude target compound. Purification was carried out by column chromatography using silica gel (Silica Gel 60N, manufactured by Kanto Chemical Co. , Inc.) and chloroform as eluent, to obtain the target compound as a pale pink solid (3.59 g, yield: 83%).

### (3) 2,3-Bis(benzyloxy)benzthioamide

In anhydrous dichloromethane (4.8 mL), 2,3-bis(benzyloxy)benzamide (393 mg, 1.2 mmol) was dissolved. Lawesson's reagent (291 mg, 0.719 mmol) was added to the obtained solution at room temperature, and the mixture was heated under reflux for 10 hours. Then, the reaction mixture was concentrated under reduced pressure to obtain a crude target compound. Purification was carried out by column chromatography using silica gel (Silica Gel 60N, manufactured by Kanto Chemical Co. , Inc.) and chloroform as eluent, to obtain the target compound as a yellow solid (371 mg, yield: 86%).

### (4) Ethyl 2-{2,3-bis(benzyloxy)phenyl}thiazole-4-carboxylate

In ethanol (5.3 mL), 2,3-bis(benzyloxy)benzthioamide (371 mg, 1.06 mmol) was dissolved. Ethyl bromopyruvate (160 µL, 1.27 mmol) was added to the obtained solution at room temperature, and the mixture was heated under reflux for 4 hours. The reaction mixture was cooled to room temperature. A saturated aqueous sodium hydrogencarbonate solution (10 mL) was added thereto, and then extraction was carried out three times using ethyl acetate (50 mL). The organic layer was dried using anhydrous magnesium sulfate, and filtrated. The filtrate was concentrated under reduced pressure to obtain a crude target compound. Purification was carried out by column chromatography using silica gel (Silica Gel 60N, manufactured by Kanto Chemical Co. , Inc.) and chloroform as eluent, to obtain the target compound as a purple (432 mg, yield: 92%).

### (5) 2-{2,3-Bis(benzyloxy)phenyl}thiazole-4-carboxylic acid

In methanol (9.7 mL), ethyl 2-{2,3-bis(benzyloxy)phenyl}thiazole-4-carboxylate (432 mg, 0.97 mmol) was dissolved. To the obtained solution, 4N sodium hydroxide aqueous solution (2.43 mL, 9.7 mmol) was added at room temperature. The mixture was stirred for 3 hours. Then, the reaction mixture was concentrated to about 10 mL under reduced pressure, and 6N hydrochloric acid (10 mL) was added thereto. Extraction was carried out three times using chloroform (50 mL). The organic layer was dried using anhydrous magnesium sulfate, and filtrated. The filtrate was concentrated under reduced pressure to obtain the target compound as a brown amorphous solid (324 mg, yield: 80%).

### Example 2: (R)-2-{2,3-Bis(benzyloxy)phenyl}-4,5-dihydrothiazo le-4-carboxylic acid

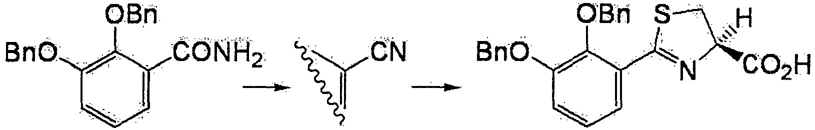

### (1) 2,3-Bis(benzyloxy)benzonitrile

In a mixed solvent of water : acetonitrile = 1 : 1 (40 mL), 2,3-bis(benzyloxy)benzamide (800 mg, 2.4 mmol) was dissolved. Palladium chloride (64 mg, 0.36 mmol) was added to the obtained solution at room temperature, and the mixture was stirred at 50°C for 6 hours. Then, the reaction mixture was concentrated to about 10 mL under reduced pressure, and extraction was carried out three times using ethyl acetate (100 mL). The organic layer was dried using anhydrous magnesium sulfate, and filtrated. The filtrate was concentrated under reduced pressure to obtain a crude target compound. Purification was carried out by column chromatography using silica gel (Silica Gel 60N, manufactured by Kanto Chemical Co., Inc.) and a mixed solution of n-hexane : ethyl acetate = 5 : 1 as eluent, to obtain the target compound as a pale yellow solid (692.3 mg, yield: 91%).

### (2) (R)-2-{2,3-Bis(benzyloxy)phenyl}-4,5-dihydrothiazole-4-ca rboxylic acid

In a mixed solvent of 1/15 M phosphate buffer solution (pH 6.4) : methanol = 1 : 1 (28 mL), 2,3-bis(benzyloxy)benzonitrile (624 mg, 1.98 mmol) was dispersed. To the obtained dispersion, L-cysteine hydrochloride monohydrate (469 mg, 2.97 mmol) and sodium hydrogencarbonate (286 mg, 3.4 mmol) were added at room temperature. The mixture was stirred at 65°C for 20 hours. The reaction mixture was cooled with ice, and filtered. The residue was washed using water (10 mL), ethanol (10 mL) and diethyl ether (10 mL) to obtain the target compound as a yellow solid (185 mg, yield: 22%).

### Example 3: 2,3-Dihydroxybenzonitrile

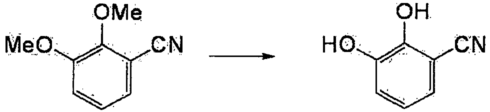

In anhydrous dichloromethane (12.26 mL), 2,3-dimethoxybenzonitrile (2 g, 12.26 mmol) was dissolved. The obtained solution was cooled to -78°C. Boron tribromide (1M dichloromethane solution, 30.64 mL, 30.64 mmol) was added thereto at the same temperature under argon atmosphere. The mixture was stirred at room temperature for 19.5 hours. The reaction mixture was cooled with ice, and water (20 mL) was added thereto. Extraction was carried out using chloroform (100 mL) and ethyl acetate (100 mL) respectively three times. The obtained organic layer was dried using anhydrous magnesium sulfate, and filtrated. The filtrate was concentrated under reduced pressure to obtain a crude target compound. Purification was carried out by column chromatography using silica gel (Silica Gel 60N, manufactured by Kanto Chemical Co., Inc.) and a mixed solution of n-hexane : ethyl acetate = 1 : 1 as eluent, to obtain the target compound as a pale brown solid (1.62 g, yield: 98%).

### Example 4: 2-(2,3-Dihydroxyphenyl)thiazole-4-carboxylic acid

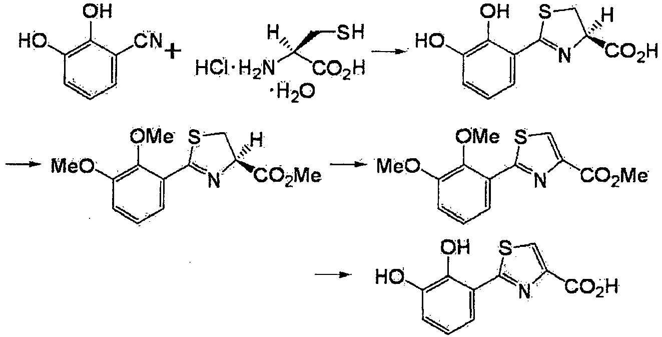

### (1) (R)-2-(2,3-Dihydroxyphenyl)-4,5-dihydrothiazole-4-carboxy lie acid

In a mixed solvent of 1/15 M phosphate buffer (pH 6.4) : methanol = 1 : 1 (151 mL), 2,3-dihydroxybenzonitrile (1.43 g, 10.58 mmol) was dispersed. To the obtained dispersion, L-cysteine hydrochloride monohydrate (2.5 g, 15.87 mmol) and sodium hydrogencarbonate (1.53 g, 18.20 mmol) were added at room temperature. The mixture was stirred at 65°C for 20 hours. The reaction mixture was cooled to room temperature, and the solvent was removed under reduced pressure. The residue was washed with ethyl acetate (50 mL), 0.5 M hydrochloric acid (20 mL) was added thereto, and extraction was carried out three times with ethyl acetate (100 mL). The organic layer was dried using anhydrous magnesium sulfate, and filtrated. The filtrate was concentrated under reduced pressure to obtain the target compound as a yellow solid (2.231 g, yield: 88%).

### (2) Methyl (R)-2-(2,3-Dimethoxyphenyl)-4,5-dihydrothiazole-4-carboxylate

In acetone (4.5 mL), (R)-2-(2,3-dihydroxyphenyl)-4,5-dihydrothiazole-4-carboxylic acid (530 mg, 2.215 mmol) was dissolved. To the obtained solution, dimethyl sulfate (723 µL, 7.64 mmol) and potassium carbonate (1 g, 7.64 mmol) were added at room temperature. The mixture was stirred at 70°C for 24 hours. The reaction dispersion was filtered using Celite, and then the filtrate was concentrated under reduced pressure to obtain a crude target compound. Purification was carried out by column chromatography using silica gel (Silica Gel 60N, manufactured by Kanto Chemical Co. , Inc.) and a mixed solution of n-hexane : ethyl acetate = 10 : 1 as eluent, to obtain the target compound as a pale yellow oil (199 mg, yield: 32%).

### (3) Methyl 2-(2,3-dimethoxyphenyl)thiazole-4-carboxylate

In 1,4-dioxane (3.5 mL), methyl (R)-2-(2,3-dimethoxyphenyl)-4,5-dihydrothiazole-4-carboxylate (199 mg, 0.707 mmol) was dissolved. To the obtained solution, manganese dioxide (1.2g, 14.15 mmol) was added at room temperature. The mixture was stirred at room temperature for 25 hours. The reaction dispersion was filtered using Celite, and then the filtrate was concentrated under reduced pressure to obtain the target compound as a white solid (193.3 mg, yield: 98%).

### (4) 2-(2,3-Dihydroxyphenyl)thiazole-4-carboxylic acid

In anhydrous dichloromethane (0.691 mL), methyl 2-(2,3-dimethoxyphenyl)thiazole-4-carboxylate (193 mg, 0.691 mmol) was dissolved. The obtained solution was cooled down to -78°C. Boron tribromide (1 M dichloromethane solution, 2.1 mL, 2.073 mmol) was added dropwise thereto under argon atmosphere at the same temperature, and the mixture was stirred at room temperature for 1 hour. Then, the reaction mixture was cooled, water (5 mL) was added thereto, and then extraction was carried out five times with ethyl acetate (50 mL). The organic layer was dried using anhydrous magnesium sulfate, and filtrated. The filtrate was concentrated under reduced pressure to obtain the target compound as a yellow solid (153.2 mg, yield: 93%).

### Example 5: Methyl 3,4,5-trihydroxybenzoate

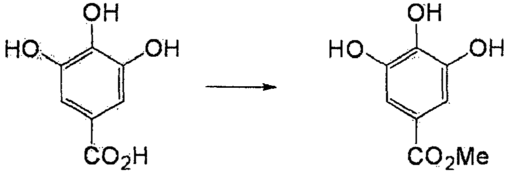

In methanol (4 mL), 3,4,5-trihydroxybenzoic acid (400 mg, 2.35 mmol) was dissolved. The obtained solution was cooled to 0°C. Concentrated sulfuric acid (0.4 mL) was slowly added to the solution, and then the mixture was heated to reflux for 17 hours. The reaction mixture was concentrated under reduced pressure, water (10 mL) was added thereto, and then extraction was carried out three times with ethyl acetate (50 mL). The organic layer was dried using anhydrous sodium sulfate, and filtrated. The filtrate was concentrated under reduced pressure to obtain the target compound as a white solid (407 mg, yield: 94%).

### Example 6: 3,4,5-Trihydroxybenzamide

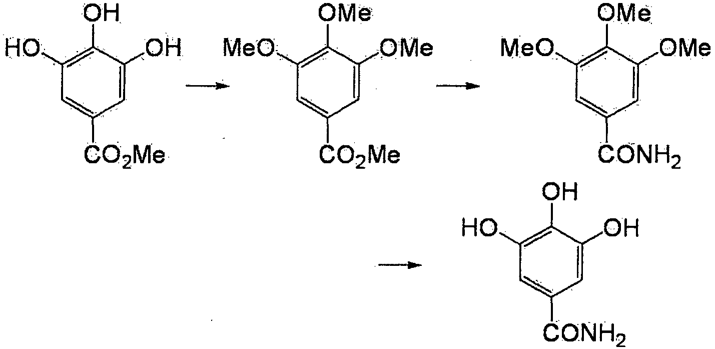

### (1) Methyl 3,4,5-trimethoxybenzoate

In acetone (6 mL), 3,4,5-trihydroxybenzoic acid (500 mg, 2.94 mmol) was dissolved. Potassium carbonate (1.8 g, and 13.2 mmol) and dimethyl sulfate (1.25 mL, 13.2 mmol) were added to the obtained solution, and the mixture was heated to reflux for 13.5 hours. The reaction dispersion was filtered using Celite, and then the filtrate was concentrated under reduced pressure to obtain a crude target compound. Purification was carried out by column chromatography using silica gel (Silica Gel 60N, manufactured by Kanto Chemical Co., Inc.) and a mixed solution of chloroform : methanol = 30 : 1 as eluent, to obtain the target compound as a white solid (630 mg, yield: 95%).

### (2) 3,4,5-Trimethoxybenzamide

To methyl 3,4,5-trimethoxybenzoate (300 mg, 1.33 mmol), 28% ammonia aqueous solution (3.8 mL) was added at room temperature. The mixture was stirred at 50°C for 10 hours. Then, the reaction mixture was concentrated under reduced pressure to obtain a crude target compound. Purification was carried out by column chromatography using silica gel (Silica Gel 60N, manufactured by Kanto Chemical Co., Inc.) and a mixed solution of chloroform : methanol = 10 : 1 and n-hexane / ethyl acetate = 1 : 2 → 0 : 100 as eluent, to obtain the target compound as a white solid (175 mg, yield: 63%).

### (3) 3,4,5-Trihydroxybenzamide

In anhydrous dichloromethane (0.45 mL), 3,4,5-trimethoxybenzamide (95 mg, 0.45 mmol) was dissolved. The obtained solution was cooled down to -78°C. Boron tribromide (1 M dichloromethane solution, 1.35 mL, 1.35 mmol) was added dropwise thereto under argon atmosphere at the same temperature, and the mixture was stirred at room temperature for 18 hours. The reaction mixture was cooled with ice, water (5 mL) was added thereto, and then extraction was carried out three times with ethyl acetate (50 mL). The organic layer was dried using anhydrous sodium sulfate, and filtrated. The filtrate was concentrated under reduced pressure to obtain a crude target compound. Purification was carried out by column chromatography using silica gel (Silica Gel 60N, manufactured by Kanto Chemical Co. , Inc.) and a mixed solution of ethyl acetate : methanol = 100 : 0 → 10 : 1 → 5 : 1 as eluent, to obtain the target compound as a pale brown solid (27.4 mg, yield: 36%).

### Example 7: Experiment to induce differentiation of mouse ES cell (1) Induction of differentiation of mouse ES cell

A culture medium to induce embryoid bodies (4 mL) was obtained by mixing an ES medium and an NP medium of which compositions are demonstrated in Table 1 in a ratio of 1 : 1. The culture medium was added to a petri dish having a diameter of 60 mm. Further, 1 × 10⁶ mouse ES cells (AB2-2) which were obtained from Dr. Alan Bradley in The Wellcome Trust Sanger Institute were added thereto, and incubated under 5% CO₂ atmosphere at 37°C for 1 day. Then, the cells were separated using a glass pipet and added to the same petri dish again, and incubated under 5% CO₂ atmosphere at 37°C for 3 days to form embryoid bodies. The formed masses of the cells were obtained, settled, and then dispersed in an NP medium (10 mL) of which composition is demonstrated in Table 1.

**Table 1**

| ES medium | | NP medium | | Differentiation medium | |
|---|---|---|---|---|---|
| DM EM /F12 (basal medium) | | DM EM /F12 (basal medium) | | DMEM/F12 (basalmedium) | |
| | 20% FBS | | 25 *µ*g/mL insulin | | 10% FBS |
| | nucleic acid | | 50 *µ*g/mL transferrin | | nucleic acid |
| | non-natural amino acid | | 20mM progesterone | | non-natural amino acid |
| | 2-mercaptoethanol | | 100*µ*M putrescine | | |
| | leukemia inhibitory factor | | 30nM sodium selenate | | |
| | | | 1 *µ*g/mL laminin | | |
| | | | 10ng/mL bFG F | | |

Then, the above cultured cell dispersion (100 µL) was added to 96 well plate which was coated with collagen type I, and was incubated under 5% CO₂ atmosphere at 37°C overnight. Separately, each examined compound was dissolved in a culture medium which was obtained by removing 2-mercaptomethanol and a leukemia inhibitory factor from an ES culture medium and which was used for inducing a differentiation, and the solutions were added each well so that the final concentrations were 25 to 100 µM. As examined compounds, compounds which were synthesized by similar methods to Examples 1 to 6 in addition to the compounds synthesized in Examples 1 to 6 were used. The structures of the examined compounds are demonstrated in Table 2. In addition, fetal bovine serum was added at a final concentration of 5% and bFGF: basic fibroblast growth factor was added at a final concentration of 2 ng/mL to each well, and the cells were cultured in an adhesive state under 5% CO₂ atmosphere at 37°C for 4 days.

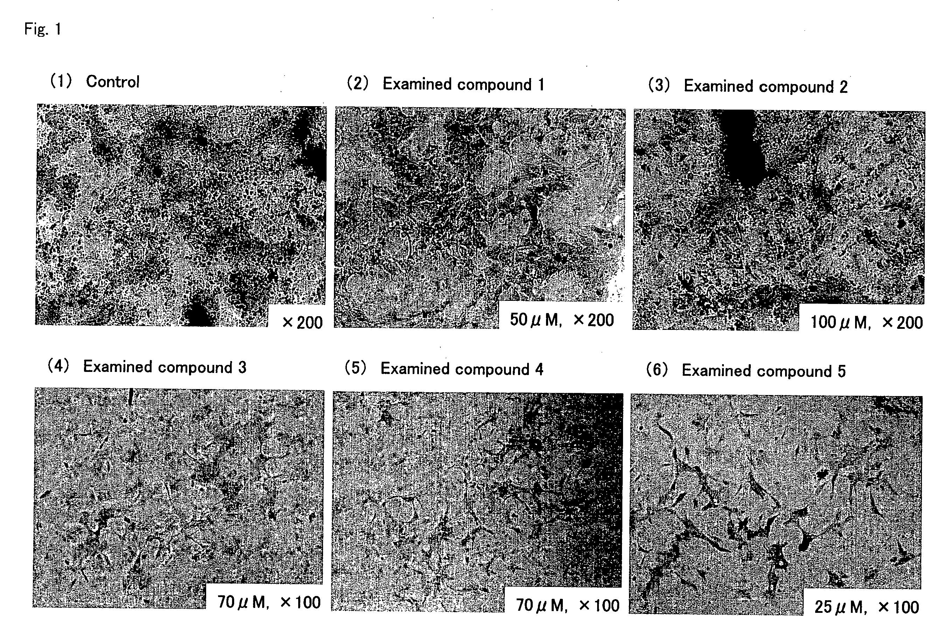

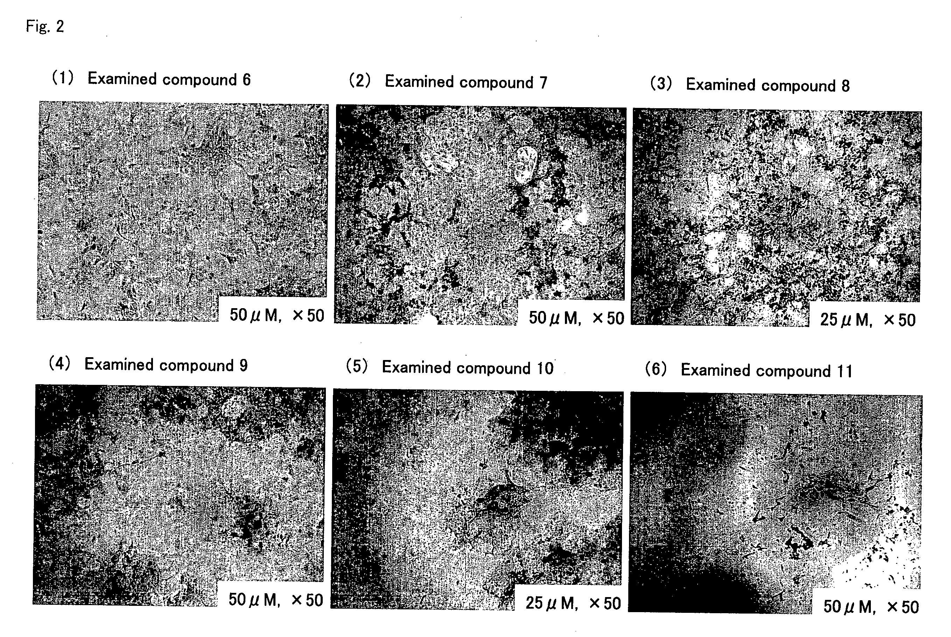

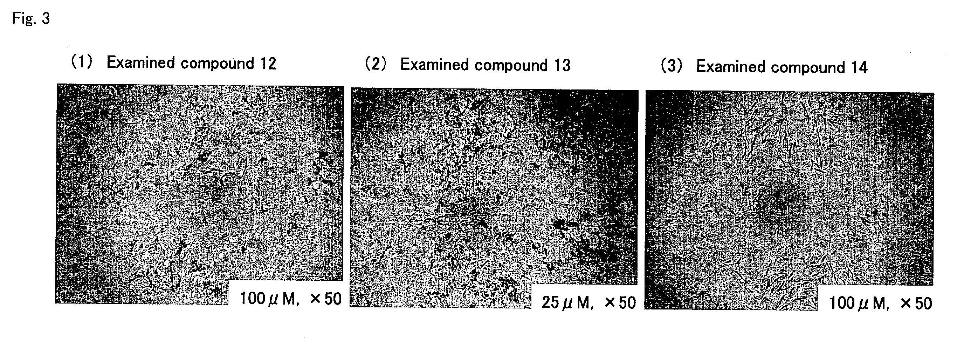

After the above culture, paraformaldehyde was added to each well at a final concentration of 2% to fix the cells. The cells were stained with hematoxylin, and the change of the morphology thereof was observed using an optical microscope. As control, only dimethylsulfoxide was added at a final concentration of 0.5% without adding the examined compound. The optical micrograph of 200 magnification of control is demonstrated as Figure 1(1), and the optical micrograph of 50, 100 or 200 magnification of the case where the change of the morphology was observed by adding each examined compound at the minimum concentration were demonstrated as Figure 1(2) to Figure 3. In addition, the minimum concentrations of each examined compound at which the change of the morphology was observed are summarized in Table 2.

**Table 2**

| Number | Catechol part | Sustituent part | Minimum inhibitory concentration |
|---|---|---|---|
| 1 | 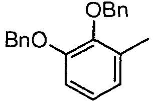 | 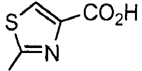 | 50 *µ*M |
| 2 | | 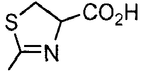 | 100 *µ*M |
| 3 | 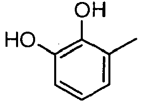 | 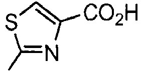 | 70 *µ*M |
| 4 | | 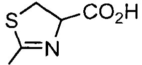 | 70 *µ*M |
| 5 | | -CN | 25 *µ*M |
| 6 | | -C(=O)NH₂ | 50 *µ*M |
| 7 | | -CO₂Me | 50 *µ*M |
| 8 | | -C(=O)NH₂ | 25 *µ*M |
| 9 | 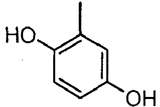 | -CO₂Me | 50 *µ*M |
| 10 | | -C(O)NH₂ | 25 *µ*M |
| 11 | 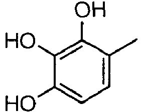 | -CO₂Me | 50 *µ*M |
| 12 | 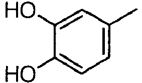 | -C(=O)NH₂ | 100 *µ*M |
| 13 | 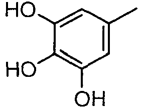 | -CO₂Me | 25 *µ*M |
| 14 | | -C(=O)NH₂ | 100 *µ*M |

As Figures 1 to 3 and Table 1, it was experimentally demonstrated that a differentiation of a mouse ES cell into a cell which has morphology with cell process can be induced by the cell differentiation inducer of the present invention at a relatively low concentration. In particular, the morphology of almost all cells could be remarkably changed by the examined compounds Nos. 12 and 13.

### (2) Identification of cell of which morphology was changed

The cell of which differentiation was induced using the examined compound 1 was fixed with paraformaldehyde as the above (1), and then washed with PBS. The cell was subjected to blocking treatment by adding a mixture of 5% goat serum / 1% BSA / PBS at a final concentration of 100 µL/well and being incubated at room temperature for 60 minutes. Then, anti-Nestin mouse IgG (manufactured by American Research Products Inc., final concentration: 5 µg/mL) or anti-Flkl rabbit IgG (manufactured by Millipore Corporation, final concentration: 5 µg/mL) was added, and the cell was incubated at 4°C overnight. Each well was washed with PBS - 0.1% Tween 20, and then anti mouse antibody labeled by FITC (Biosource International, Inc., final concentration: 1 µg/mL) or rabbit IgG antibody (Biosource International, Inc., final concentration: 1 µg/mL) was added as a secondary antibody. The cell was incubated at room temperature for 2 hours. Each well was washed with PBS - 0.1% Tween 20, and then fluorographies were taken using IN Cell Analyzer 1000 (manufactured by GE Gelthcare).

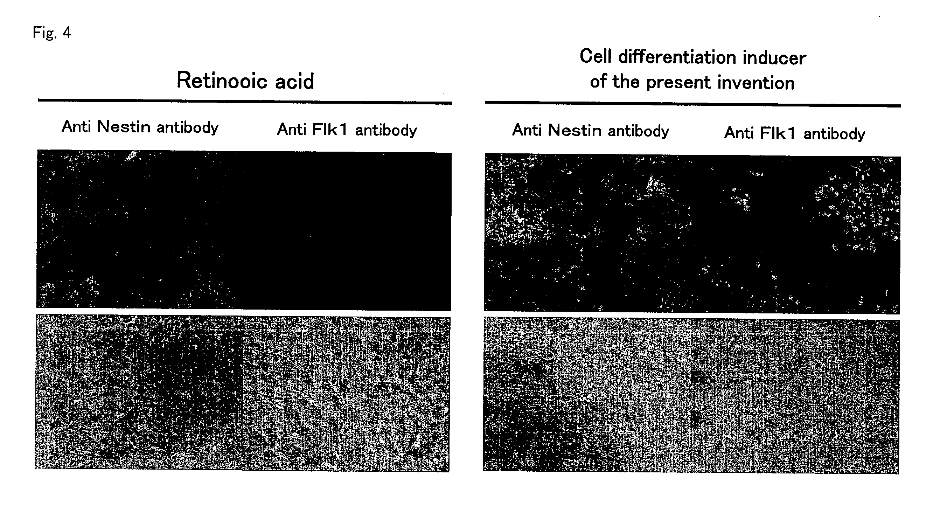

In addition, a similar treatment was carried out except that 1 µM of retinoic acid, which has a function to induce a differentiation of an undifferentiated cell into a neuroectoderm, was used. Among the obtained photographs, the photographs in the case where retinoic acid was used are demonstrated in the first and second columns from the left of Figure 4, the photographs in the case where the cell differentiation inducer of the present invention was used are demonstrated in the first and second columns from the right of Figure 4, the photographs in the case where anti mouse antibody labeled by FITC was detected are demonstrated in the upper row of Figure 4, and the photographs in the case where rabbit IgG antibody was detected are demonstrated in the lower row of Figure 4.

As Figure 4, in the cell of which differentiation was induced using retinoic acid, Nestin was expressed but Flk1 was not expressed. Since Nestin is a marker of an ectodermal stem cell and Flk1 is a marker of a mesodermal stem cell, it could be confirmed that a differentiation of an undifferentiated cell into an ectodermal stem cell can be induced by retinoic acid. On the other hand, the cell of which differentiation was induced by the cell differentiation inducer of the present invention was positive for Nestin and Flk1. From the experimental result, it was clarified that the cell differentiation inducer of the present invention can induce a differentiation of an undifferentiated cell into a neural crest cell, which falls into the classification of an ectodermal cell but also has properties of a mesodermal cell.

### (3) Gene expression analysis by RT-PCR

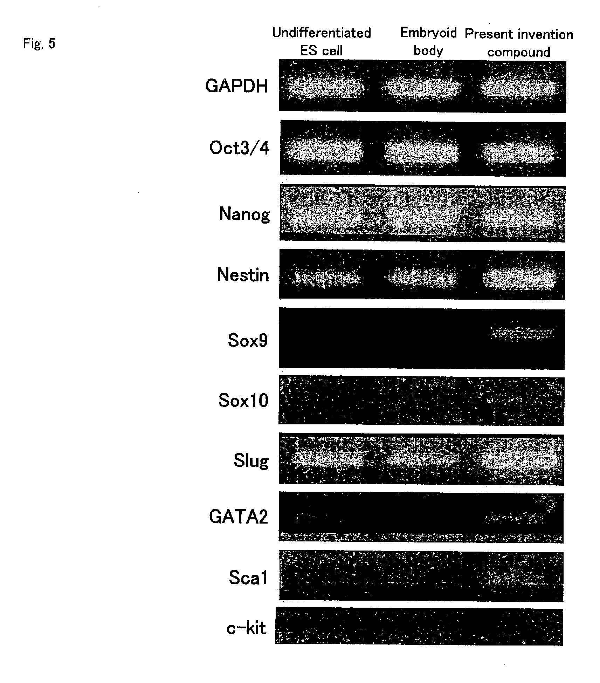

Differentiation of a mouse ES cell was induced similarly to the above (1) except that the cell was treated with 80 µM of the examined compound 4 for 5 days. From the obtained cell, total RNA was extracted using TRIzol reagent. Using the total RNA, cDNA was synthesized by an ordinary method. A polymerase chain reaction was carried out using the synthesized cDNA as a template and each primer of Oct3/4· Nanog - a marker of an undifferentiated cell, Nestin - a marker of a neural stem cell, Sox9· Sox10· Slug - a marker of a neural crest cell, GATA2 - a marker of a mesoderm, Sca-1 - a marker of a stem cell, and c-kit - a marker of a hematopoietic stem cell for analyzing the expression of each marker. In addition, for comparison, the expression was similarly analyzed with respect to an undifferentiated mouse ES cell and an embryoid body obtained by culturing a mouse ES cell for 3 days. The result is demonstrated in Figure 5.

As Figure 5, there is almost no difference of gene expression between the mouse ES cell and the embryoid body thereof. In addition, in the mouse ES cell and the embryoid body thereof, Sox9 and Sox10, which are markers of a neural crest cell, were not expressed. On the other hand, in the mouse ES cell which was treated with the cell differentiation inducer of the present invention, Sox9 and Sox10 were expressed. In addition, Slug, which is similarly a marker of a neural crest cell, was remarkably expressed. From the experimental result, it was similarly clarified that the cell differentiation inducer of the present invention can induce a differentiation of an undifferentiated cell into a neural crest cell.

### Example 8: Experiment to induce differentiation of human iPS cell

A differentiation of a human iPS cell (obtained from Institute of Physical and Chemical Research (Japan), hPS0001 201B7 lot3) instead of a mouse ES cell was induced similarly to Example 7(1).

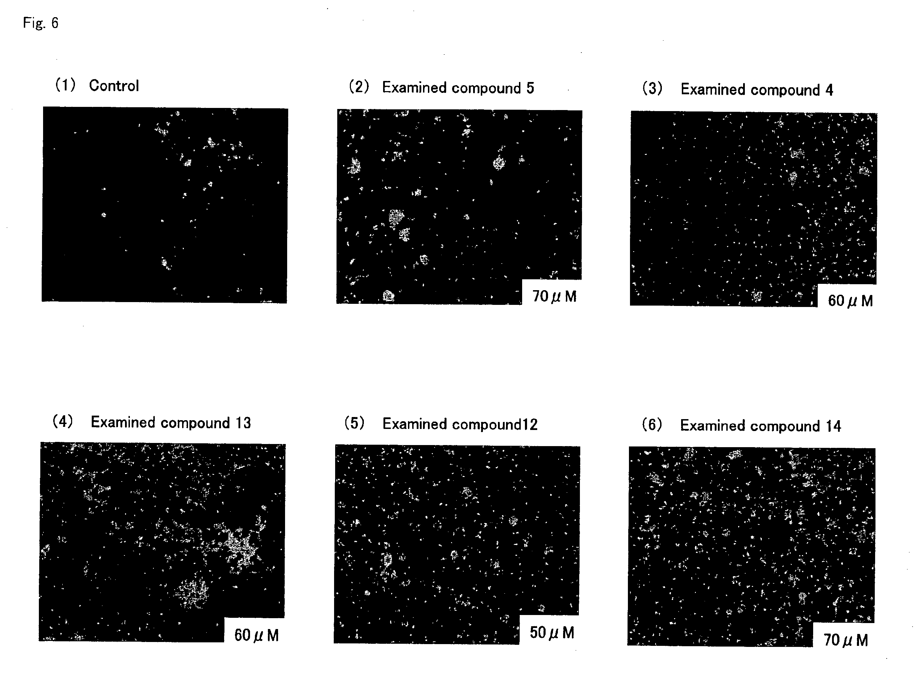

As described above, the cell of which differentiation was induced using the examined compound was fixed with paraformaldehyde, and then washed with PBS. The cell was subjected to blocking treatment by adding a mixture of 5% goat serum / 1% BSA / PBS at a concentration of 100 µL/well and being incubated at room temperature for 60 minutes. Then, anti-Nestin mouse IgG (manufactured by Sigma, final concentration: 5 µg/mL) was added, and the cell was incubated at 4°C overnight. Each well was washed with PBS - 0.1% Tween 20, and then anti mouse antibody labeled by FITC (Biosource International, Inc., final concentration: 1 µg/mL) was added as a secondary antibody. The cell was incubated at room temperature for 2 hours. In addition, the cell was stained using Hoechst 33258. Each well was washed with PBS - 0.1%. Tween 20, and then fluorographies were taken using IN Cell Analyzer 1000 (manufactured by GE Gelthcare). The result of image analysis was demonstrated in Figure 6. In Figure 6, the green portion represents the cell which is positive for Nestin, which is a marker of an ectodermal stem cell, and blue portion represents the cell which is positive for Hoechst 33258, which is a nucleus staining agent.

As Figure 6, in control, the number of the cell itself was small and the ratio of Nestin-positive cell relative to the total cells was also clearly small. On the other hand, when the cell differentiation inducer of the present invention was used, the cells were wholly positive for Nestin sufficiently, and it was confirmed that the cells were differentiated into ectodermal stem cells. From the result, the differentiation-inducing effect of the present invention compound was experimentally demonstrated even in a human iPS cell. In addition, there is slippage between Hoechst33258-positive cell and Nestin-positive cell. It is thought that this is because the intensity of green of the cells was increased in the upper part of the photographs due to the auto-contrast of the image analysis soft, and the intensity of staining of the cells itself was decreased since the cell process of the cells were intensely stained by Nestin.

### Example 9: Combinational effect with retinoic acid

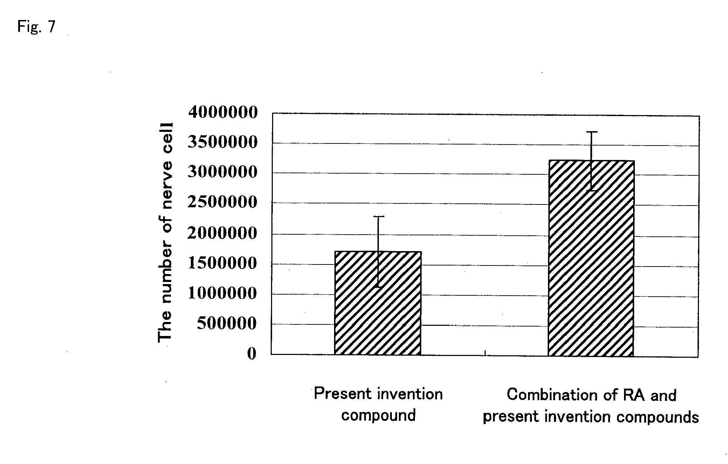

A culture medium to induce an embryoid (4 mL) was obtained by mixing an ES medium and an NP medium of which compositions are demonstrated in Table 1 in a ratio of 1 : 1. The culture medium was added to a petri dish having a diameter of 60 mm. Further, 1 × 10⁶ mouse ES cells (AB2-2) which were obtained from Dr. Alan Bradley in The Wellcome Trust Sanger Institute were added thereto, and incubated under 5% CO₂ atmosphere at 37°C for 1 day. Then, the cells were separated using a glass pipet and added to the same petri dish again, and incubated under 5% CO₂ atmosphere at 37°C for 3 days to form embryoid bodies. The formed masses of the cells were obtained, settled, and then dispersed in an NP medium (10 mL) of which composition is demonstrated in Table 1. Then, the above cultured cell dispersion (5 mL) was added to 96 well plate which was coated with collagen type I, and was incubated under 5% CO₂ atmosphere at 37°C overnight. On the next day, the examined compound 5 only or with retinoic acid in combination in each final concentration of 10 nM was added thereto, and the cells were incubated in adhesive state under 5% CO₂ atmosphere at 37°C for 4 days. After the incubation, the cells were washed with PBS, and separated using 0.25% Trypsin. The number of the cells was counted. This experiment was carried out four times, and the average of the counted numbers was obtained. The result was demonstrated in Figure 7. In the Figure 7, "RA" represents retinoic acid.

As Figure 7, the number of the nervous system cells in a case where the present invention compound was used was 1.71±0.51×10⁶; on the other hand, the number was remarkably increased in a case where retinoic acid was used in combination as 3.23±0.42×10⁶ and became about 1.89 times. The P value in the case was 0.0023. From the result, it was clarified that when the present invention compound is used in combination with retinoic acid, differentiation-inducing effect into a nervous system cell can be further improved.

### Example 10: Experiment to induce differentiation of mouse ES cell

A culture medium to induce an embryoid bodies used in Example 7 (4 mL) was added to a petri dish having a diameter of 60 mm. Further, 1 × 10⁶ mouse ES cells (AB2-2) were added thereto, and incubated under 5% CO₂ atmosphere at 37°C for 3 days. The obtained embryoid was added into a 15 mL volume tube. The above petri dish was washed by adding lxPBS (5 mL), and the PBS was added into the above tube. The half quantity of the obtained cells was transferred to a separate tube, and the tube was stood still at room temperature for 5 minutes. After the supernatant was removed, an NP medium (10.5 mL) used in Example 7 was added. The cells were dispersed well, and the mixture was added to each well of a 96 well plate which was coated with collagen in an amount of 100 µL. The cells were incubated under 5% CO₂ atmosphere at 37°C overnight. To each well, a proper amount of a differentiation medium of which composition is demonstrated in Table 3 was added.

**Table 3**

| Differentiation medium | |
|---|---|
| DMEM/F12 (basal medium) | |
| | 5% FBS |
| | 50 *µ*g/mL insulin |
| | 50 *µ*g/mL transferrin |
| | 20mM progesterone |
| | 30mM sodium selenate |
| | 1 *µ*g/mL laminin |
| | 10ng/mL bFG F |

In DMSO, 3,4-dihydroxybenzonitrile (manufactured by Tokyo Chemical Industry Co. , Ltd.) was dissolved. The solution was added to each well so that the total amount became 200 µL in a final concentration of 12.5 µM, 25 µM or 50 µL. Further, blood serum and bFGF: basic fibroblast growth factor were added to each well respectively in final concentrations of 5% and 10 ng/mL, and the cells were cultured under 5% CO₂ atmosphere at 37°C for 4 days.

After the cultivation, 100 µL of the culture medium was removed from each well. Then, paraformaldehyde was added in a final concentration of 4%, and the cells were fixed by standing still overnight. Next, paraformaldehyde was removed. For washing procedure, PBS (200 µL) was added to each well, the wells were stood still for 3 minutes, and PBS was removed. This washing procedure was repeated 10 times. The cells were stained by adding hematoxylin (60 µL) to the wells. For washing procedure, PBS (200 µL) was added to each well, the wells were stood still for 3 minutes, and PBS was removed. This washing procedure was repeated 10 times. After PBS (100 µL) was added to each well, the change of the cells' morphology was observed using an optical microscope.

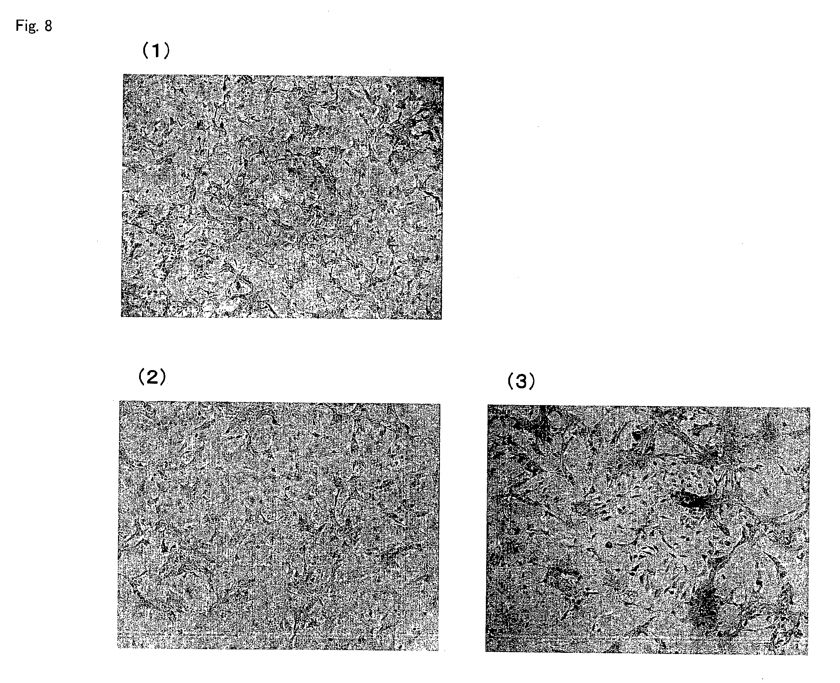

The optical micrograph of 50 magnification of the case where the present invention compound was added in a concentration of 50 µM was demonstrated as Figure 8(1), and the optical micrographs of 100 magnification were demonstrated as Figures 8(2) and 8(3). As Figure 8, nervous system cells which had cell processes all around were observed, and a cell which had other forms was not observed. Therefore, it was experimentally demonstrated that a differentiation of an undifferentiated cell into a nervous system cell can be induced by the present invention compound with high selectivity.

## Claims

1. A cell differentiation inducer, comprising a catechol derivative represented by the following formula (I): 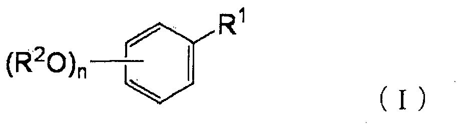 wherein
R¹ is a carboxy group, a (C₁₋₆ alkoxy) carbonyl group, a C₁₋₇ alkanoyl group, a C₂₋₈ alkynyl group, a carbamoyl group, a cyano group, a nitro group, a halogen atom, an oxazolyl group optionally-having a substituent α, a thiazolyl group optionally-having a substituent α, an oxazolinyl group optionally-having a substituent α, or a thiazolinyl group optionally-having a substituent α;
R² is a hydrogen atom, a C₁₋₆ alkyl group or a benzyl group;
n is an integer of not less than 2 and not more than 5;
substituent α is one or more groups selected from a carboxy group, a (C₁₋₆ alkoxy) carbonyl group, a C₁₋₇ alkanoyl group, a carbamoyl group, a cyano group, a nitro group and a halogen atom;
provided that two or more R²O groups may be the same or different from each other.

2. The cell differentiation inducer according to claim 1, wherein R² is a hydrogen atom.

3. The cell differentiation inducer according to claim 1 or 2, wherein two or more R²O groups are adjacent to each other.

4. The cell differentiation inducer according to any one of claims 1 to 3, wherein R¹ is a cyano group or a carbamoyl group.

5. The cell differentiation inducer according to anyone of claims 1 to 4, further comprising retinoic acid.

6. The cell differentiation inducer according to any one of claims 1 to 5, used for inducing a differentiation of an undifferentiated cell into a nervous system cell.

7. The cell differentiation inducer according to any one of claims 1 to 5, used for inducing a differentiation of an undifferentiated cell into a neural crest cell.

8. A culture medium, comprising the cell differentiation inducer according to any one of claims 1 to 5.

9. A catechol derivative represented by the following formula (I), used for inducing a differentiation of an undifferentiated cell: 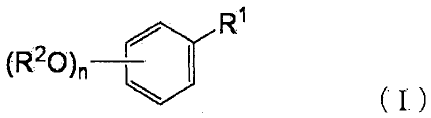 wherein
R¹ is a carboxy group, a (C₁₋₆ alkoxy) carbonyl group, a C₁₋₇ alkanoyl group, a C₂₋₈ alkynyl group, a carbamoyl group, a cyano group, a nitro group, a halogen atom, an oxazolyl group optionally-having a substituent α, a thiazolyl group optionally-having a substituent α, an oxazolinyl group optionally-having a substituent α, or a thiazolinyl group optionally-having a substituent α;
R² is a hydrogen atom, a C₁₋₆ alkyl group or a benzyl group;
n is an integer of not less than 2 and not more than 5;
substituent α is one or more groups selected from a carboxy group, a (C₁₋₆ alkoxy) carbonyl group, a C₁₋₇ alkanoyl group, a carbamoyl group, a cyano group, a nitro group and a halogen atom;
provided that two or more R²O groups may be the same or different from each other.

10. A method for inducing a differentiation of an undifferentiated cell to a nervous system cell, comprising the step for cultivating an undifferentiated cell in a culture medium containing a catechol derivative represented by the following formula (I) : 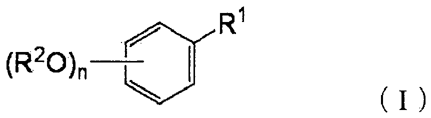 wherein
R¹ is a carboxy group, a (C₁₋₆ alkoxy) carbonyl group, a C₁₋₇ alkanoyl group, a C₂₋₈ alkynyl group, a carbamoyl group, a cyano group, a nitro group, a halogen atom, an oxazolyl group optionally-having a substituent α, a thiazolyl group optionally-having a substituent α, an oxazolinyl group optionally-having a substituent α, or a thiazolinyl group optionally-having a substituent α;
R² is a hydrogen atom, a C₁₋₆ alkyl group or a benzyl group;
n is an integer of not less than 2 and not more than 5;
substituent α is one or more groups selected from a carboxy group, a (C₁₋₆ alkoxy) carbonyl group, a C₁₋₇ alkanoyl group, a carbamoyl group, a cyano group, a nitro group and a halogen atom;
provided that two or more R²O groups may be the same or different from each other.
